# EUROPÄISCHE PATENTSCHRIFT

(11) **EP 0 514 662 B1**
(45) Veröffentlichungstag und Bekanntmachung des Hinweises auf die Patenterteilung: **03.07.1996**
(21) Anmeldenummer: 92106311.1
(22) Anmeldetag: 11.04.1992
(51) Int. Cl.: A61B 17/16

(54) **Chirurgisches Instrument zum Positionieren von osteosynthetischen Befestigungselementen, insbesondere Knochenschrauben**
Surgery device to position osteosynthesis fixation elements, particularly bone screws
Instrument chirurgical pour positionner des éléments de fixation d'ostéosynthèse en particulier les vis osseuses

(30) Priorität: 24.05.1991 CH 1543/91
(43) Veröffentlichungstag der Anmeldung: 25.11.1992
(73) Patentinhaber: Synthes AG, Chur, CH-7002 Chur (CH)
(72) Erfinder: Frigg, Robert, CH-7270 Davos-Platz (CH)
(74) Vertreter: Lusuardi, Werther Giovanni, Dr.

(56) Entgegenhaltungen:
- WO-A-92/01422
- CH-A- 668 692
- US-A- 4 911 153

## Beschreibung

Die Erfindung bezieht sich auf ein chirurgisches Instrument gemäss dem Oberbegriff von Anspruch 1.

In der operativen Frakturbehandlung von Femurfrakturen, weisen die Femurhals-Schaftfraktur-Kombinationen die grössten Probleme auf. Der Grund für diese Problematik liegt in den inkompatiblen Osteosyntheseverfahren der beiden verschiedenartigen Frakturen.

Das Dokument CH-A-668 692 betrifft einen Marknagel , der die Merkmale gemäß dem ersten Teil des Anspruchs 1 aufweist.

Bezogen auf den Stand der Technik werden Femurschaftfrakturen mit intramedullären Nägeln fixiert. Schenkelhalsfrakturen werden mit Winkelplatten, dynamischen Hüftschrauben, oder durch drei parallel eingebrachte Schrauben fixiert. Bei jüngeren Patienten wird vor allem die Verschraubung durchgeführt, da diese Art der Versorgung die wenigsten zusätzlichen Beschädigungen des Schenkelhalses mit sich bringt.

Betrachtet man die Dringlichkeit, mit welcher die beiden Frakturen fixiert werden müssen, liegt die Dringlichkeit der Versorgung der Schenkelhalsfraktur an erster Stelle. Bei dieser Fraktur besteht die Gefahr, dass wenn sie nicht fixiert wird, eine Durchblutungsstörung des Femurkopfes eintritt. Eine solche Durchblutungsstörung endet meist in der Resektion des Femurkopfes und dem Einbringen einer Totalprothese. Dieser Komplikation gegenüber, ist eine verzögerte Frakturheilung im Femurschaftbereich leichter nachzubehandeln. Dieser Tatsache zum Trotz, werden immer mehr intramedulläre Implantate zur Fixation dieser Art von Kombinationsfrakturen angewendet. Solche sogenannte Marknägel besitzen im proximalen Nagelende Querbohrungen die zur Aufnahme von Schrauben dienen, welche von lateral, durch den Nagel in den Femurkopf eingebracht werden können.

Die für die Fixation der Schenkelhalsfraktur benötigten Schrauben besitzen einen Durchmesser von mindestens 6,5 mm und werden parallel zueinander in den Femurkopf eingebracht. Da diese Schrauben, durch den Marknagel bedingt, nur untereinander eingebracht werden, können nur zwei Schrauben durch den Nagel in den Femurkopf eingesetzt werden.

Damit der Marknagel durch die beiden Querbohrungen, und der zusätzlichen Krafteinleitung über die Schrauben nicht zu stark geschwächt wird, besitzt dieser einen grösseren Durchmesser. Diese Durchmesserzunahme bedingt ein grösseres Aufbohren des Markraumes, was in keinem Verhältnis zu der zu fixierenden Fraktur im Schaftbereich steht.

Der Hauptnachteil dieses Osteosyntheseverfahrens ist jedoch, dass zuerst die Schaftfraktur versorgt wird, ohne dass die Schenkelhalsfraktur primär stabilisiert werden kann. Der Nachteil dieser Methode ist vor allem dann ersichtlich, wenn man weiss, wie gross der Kraftaufwand für das Einschlagen eines solchen Marknagels ist. Jeder für das Einschlagen des Nagels benötigte Hammerschlag, distrahiert die Fraktur im Halsbereich, was unbedingt zu einer Gefährdung der Blutversorgung im Femurkopfbereich führt.

Hier will die Erfindung Abhilfe schaffen. Die Erfindung, wie sie im kennzeichnenden Teil des Anspruchs 1 gekennzeichnet ist, löst die Aufgabe, ein chirurgisches Instrument zum Positionieren von osteosynthetischen Befestigungselementen, wie Knochenschrauben oder Knochennägel zu schaffen, mit dem die osteosynthetischen Befestigungselemente tangential neben einem Marknagel oder einem Manipuliermarknagel gesetzt werden können. Es ermöglicht insbesondere Knochenschrauben vor oder hinter den Marknagel zu setzen und zwar unter einem frei wählbaren Winkel zur Achse des Marknagels.

Die durch die Erfindung erreichten Vorteile sind im wesentlichen darin zu sehen, dass dank dem erfindungsgemässen chirurgischen Instrument trotz Anwendung eines Marknagels die Primärfixation einer Schenkelhalsfraktur ermöglicht wird, was durch eine marknagelunabhängige Verschraubung des Femurkopfes erzielt wird. Ein weiterer Vorteil ist darin zu sehen, dass Bohrungen auf jeder beliebigen Höhe in jeder beliebigen Winkelstellung bezüglich der Marknagelposition angebracht werden können und dies ohne befürchten zu müssen, dass der Marknagel getroffen wird.

Ein Ausführungsbeispiel der Erfindung, welches zugleich das Funktionsprinzip erläutert, ist in der Zeichnung dargestellt und wird im folgenden näher beschrieben.
Fig. 1 stellt eine schematische Darstellung der erfindungsgemässen Vorrichtung im demontierten Zustand für den Einsatz am Femur dar;
Fig. 2 stellt eine Ansicht von unten von der Spitze des Manipuliermarknagels mit der erfindungsgemässen Vorrichtung im zusammengesetzten Zustand dar;
Fig. 3 stellt eine seitliche Ansicht der erfindungsgemässen Vorrichtung im zusammengesetzten Zustand dar;
Fig. 4 stellt eine seitliche Ansicht der erfindungsgemässen Vorrichtung gemäss Fig. 3 mit nach oben verschwenkter Bohrlehre dar;
Fig. 5 stellt eine latero-mediale Ansicht der erfindungsgemässen Vorrichtung am Femur dar mit zwei verschiedenen Positionen der Bohrlehre;
Fig. 6 stellt eine antero-posteriore Frontalansicht der erfindungsgemässen Vorrichtung gemäss Fig. 5 dar;
Fig. 7 stellt eine antero-posteriore Ansicht der erfindungsgemässen Vorrichtung am Femur dar, die zum Setzen zweier paralleler Knochenschrauben verwendet wird;
Fig. 8 stellt eine cranio-caudale Aufsicht auf die erfindungsgemässen Vorrichtung am Femur dar mit zwei verschiedenen Positionen der Bohrlehre; und
Fig. 9 zeigt einen teilweisen antero-posterioren Schnitt durch eine abgeschlossene Osteosynthese am Femur dar.

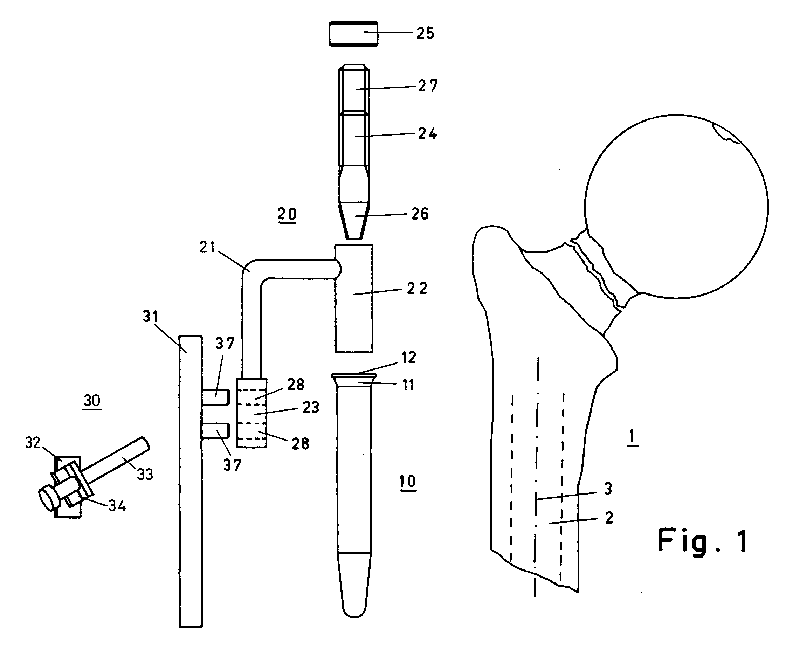

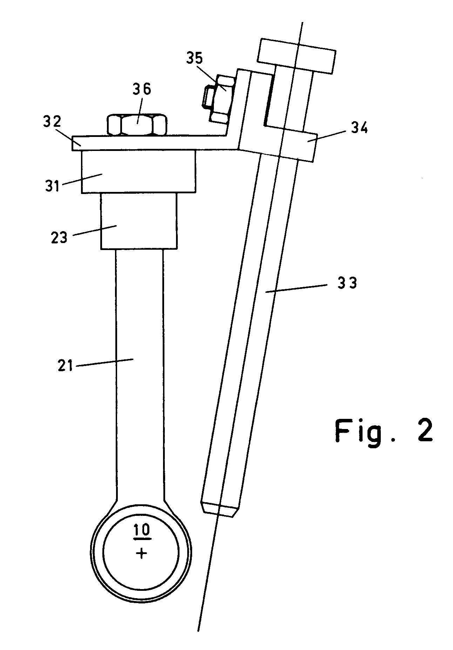

Wie aus den Figuren 1 und 2 ersichtlich besteht das erfindungsgemässe chirurgische Instruments im wesentlichen aus zwei Teilen: Einem U-förmigen Bügel (20) mit einem marknagelseitigen Ende (22) und einem bohrlehrenseitigen Ende (23), der mit seinem marknagelseitigen Ende (22) koaxial und in lösbarer Weise mit einem in den Markkanal (2) eines Röhrenknochens (1) eingeführten Manipuliermarknagel (10) verbindbar ist, sowie einer am bohrlehrenseitigen Ende (23) des Bügels (20) befestigbaren Bohrlehre (30).

Der hier in Form eines Manipuliermarknagels 10 vorliegende Marknagel weist gegenüber einem üblichen zur Implantation vorgesehenen Marknagel geringere Dimensionen auf. Typischerweise beträgt sein Aussendurchmesser maximal 12 mm und seine Länge 90 mm (oft bloss 9 mm, bzw. 80 mm). Diese Dimensionierung genügt vollständig für den angestrebten Zweck der eindeutigen Festlegung der Längsachse 3 des Markkanals 2 und zur Schaffung einer Referenzbasis für den Bügel 20 und die Bohrlehre 30. Der Manipuliermarknagel 10 weist an seinem proximalen Ende 11 Befestigungsmittel 12 - in Form einer konischen Bohrung mit Innengewinde - auf, zwecks lösbarer Befestigung des Bügels 20.

Der Bügel 20 besteht aus dem eigentlichen U-förmigen Einschlagbügel 21 mit einem marknagelseitigen Ende 22 und einem bohrlehrenseitigen Ende 23, einem konischen Gewindebolzen 24 und einer Rändelmutter 25. Das marknagelseitigen Ende 22 des Einschlagbügels 21 ist als Hohlzylinderabschnitt ausgebildet, der den konischen Gewindebolzen 24 aufnehmen kann. Das bohrlehrenseitigen Ende 23 des Einschlagbügels 21 weist Verbindungsmittel 28 zur Befestigung der Bohrlehre 30 auf. Die Verbindungsmittel 28 bestehen bei dieser Ausführungsform aus zwei Bohrungen, welche gleichzeitig als Führung und Bohrlehre zum Setzen von Verriegelungsschrauben dienen.
Der konische Gewindebolzen 24 weist an seinem vorderen Ende eine vordere Gewindepartie 26 auf, welche mit dem Innengewinde der konische Bohrung der Befestigungsmittel 12 korrespondiert. An seinem hinteren Ende weist der konische Gewindebolzen 24 eine hintere, kreiszylindrische Gewindepartie 27 auf, welche mit dem Innengewinde der Rändelmutter 25 korrespondiert.
Die Befestigung des Bügels 20 am proximalen Ende des Manipuliermarknagels 10 erfolgt dadurch, dass der konische Gewindebolzen 24 mit seiner vorderen Gewindepartie 26 in das Innengewinde der konischen Bohrung der Befestigungsmittel 12 des Manipuliermarknagels 10 eingedreht wird und hierauf das als Hohlzylinder ausgebildete, marknagelseitige Ende 22 des Bügels 20 über die hintere, kreiszylindrische Gewindepartie 27 des konischen Gewindebolzens 24 geschoben und mit der Rändelmutter 25 fixiert wird um ein axiales Verschieben des Hohlzylinders zu verhindern. Diese Fixation ist besonders wichtig, da die Bohrungen 28 im Bügel 20 mit den Verriegelungsbohrungen des später zu implantierenden Marknagels exakt übereinstimmen müssen.

Die Bohrlehre 30 besteht aus einer Längsschiene 31, einem an der Längsschiene 31 befestigbaren und längsverschieblich angeordneten Reiter 32 und einer seitlich am Reiter 32 drehbar gelagerten Bohrbüchse 33. Die Längsschiene 31 weist Befestigungsmittel 37 auf zur lösbaren Befestigung in den Bohrungen 28 am bohrlehrenseitigen Ende 23 des Bügels 20 auf.

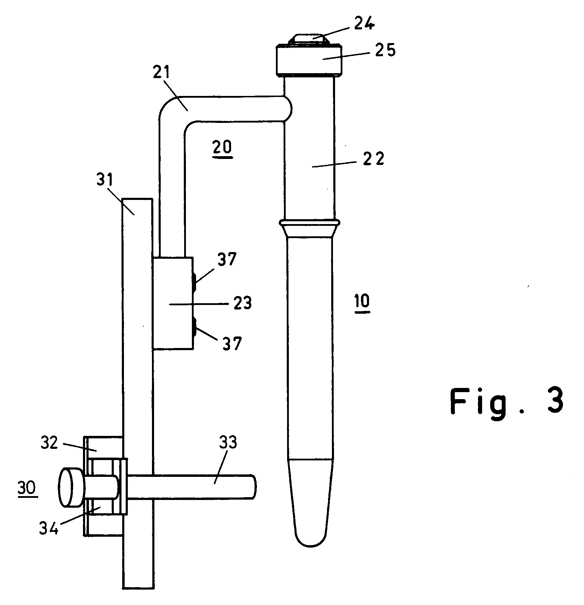

Betrachtet man die erfindungsgemässe Vorrichtung wie in Fig. 2 dargestellt von der Spitze des Manipuliermarknagels 10 her, so ist die Bohrbüchse 33 so ausgerichtet, dass sie in der Verlängerung, tangential neben dem Manipuliermarknagel 10 vorbei zielt. Der Reiter 32 kann dabei mittels einer Feststellmutter 36 an der Längsschiene 31 fixiert werden. Die drehbare Lagerung 34 der Bohrbüchse 33 kann mittels der Feststellmutter 35 am Reiter 32 fixiert werden. Nach vollständiger Montage aller Teile stellt sich die erfindungsgemässe Vorrichtung wie in Fig. 3 dar.

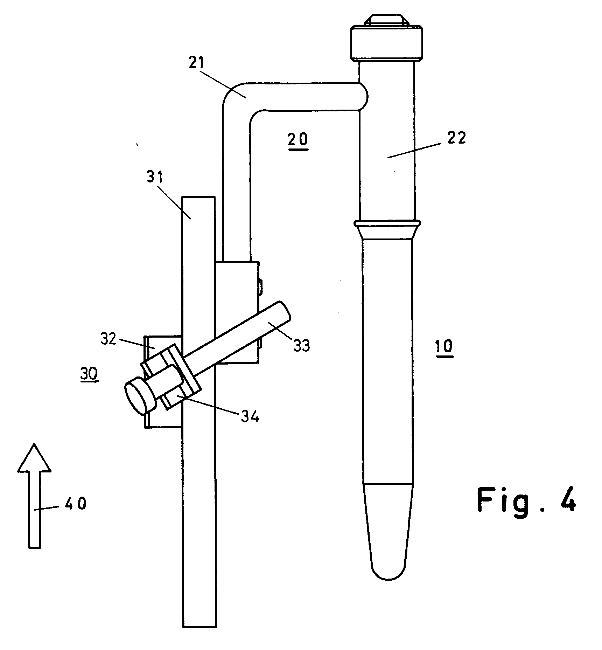

Durch Lösen der Feststellmutter 36 kann die Bohrbüchse 33 mit ihrer drehbaren Lagerung 34 beispielswiese aus Ihrer Position gemäss Fig. 3 in Richtung des Pfeiles 40 entlang der Längsschiene 31 in jede gewünschte Position, beispielsweise gemäss Fig. 4, verschoben werden und dort durch Anziehen der Feststellmutter 36 wieder fixiert werden.

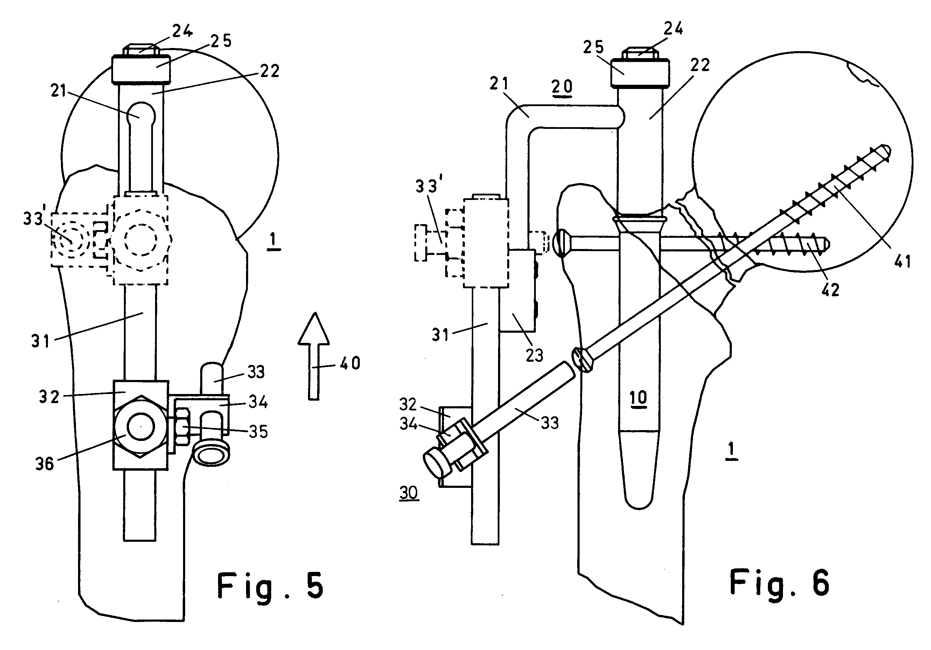

In den Fig. 5 und 6 ist dargestellt, wie mittels dem erfindungsgemässen Instrument in zwei aufeinanderfolgenden Schritten zwei Knochenschrauben 41 und 42 gesetzt werden können. Durch die geneigte Bohrlehre 33 kann die Knochenschraube 41 anterior am Manipuliermarknagel 10 vorbei in den Femurkopf gesetzt werden. Durch Lösen der Feststellmutter 36 kann die Bohrbüchse 33 durch Verschiebung des Reiters 32 in Richtung des Pfeiles 40 nach proximal verschoben werden und dort durch Anziehen der Feststellmutter 36 wieder fixiert werden. Auch die Richtung der Bohrbüchse 33 lässt sich durch Lösen und Wiederanziehen der Feststellmutter 35 je nach den Erfordernissen (hier senkrecht zum Manipuliermarknagel 10) einstellen.

Die Position der verschobenen Bohrbüchse 33' ist in den Fig. 5 und 6 punktiert dargestellt. Durch die verschobene Bohrbüchse 33' lässt sich nun eine zweite Knochenschraube 42 posterior am Manipuliermarknagel 10 vorbei in den Femurkopf setzen.

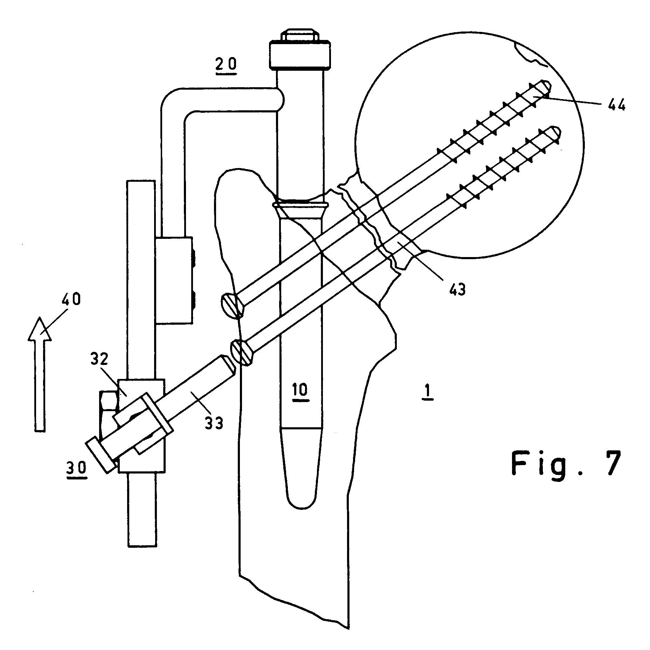

In Fig. 7 ist dargestellt, wie durch Verschiebung des Reiters 32 in Richtung des Pfeils 40 bei gleichbleibender Neigung der Bohrbüchse 33 zwei Knochenschrauben 43 und 44 parallel in einem vorgegebenen Abstand zueinander gesetzt werden können. Eine solche parallele Lage der Knochenschrauben 43 und 44 erlaubt eine maximale Kompression der Schenkelhalsfraktur.

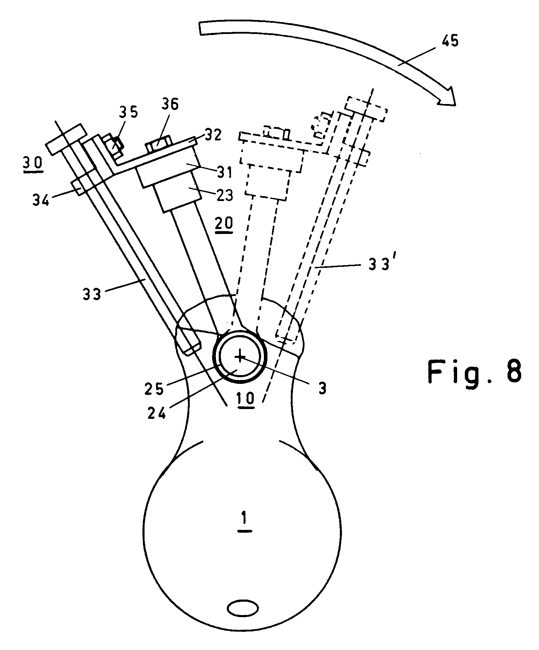

In Fig. 8 ist dargestellt, wie das erfindungsgemässe Instrument um die mit der Längsachse des Markkanals 3 zusammenfallende Achse des Manipuliermarknagels 10 in Richtung des Pfeiles 45 verschwenkt werden kann. Wird gleichzeitig die Lagerung 34 der Bohrbüchse 33 um 180° gedreht, so erreicht man die punktiert dargestellte Position 33' der Bohrbüchse. Auf diese Weise ist es möglich Knochenschrauben anterior oder posterior zum Manipuliermarknagel 10 in den Femur 1 einzusetzen.

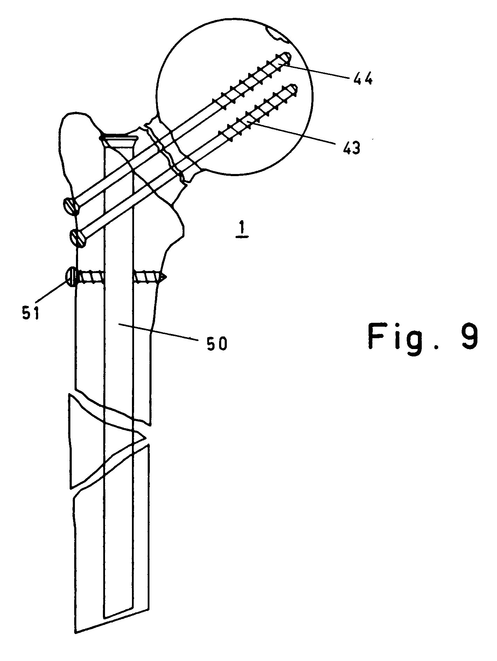

In Fig. 9 schliesslich ist eine abgeschlossene Osteosynthese dargestellt, bei welcher zwei parallele Zugschrauben 43,44 in den Kopf des Femur 1 gesetzt sind. Der Manipuliermarknagel 10 ist durch einen permanenten Marknagel 50 ersetzt worden, der proximal mit einer Schraube 51 verriegelt worden ist.

Im folgenden wird kurz die Operationstechnik bei der Versorgung einer Femurfraktur unter Anwendung des erfindungsgemässen chirurgischen Instruments beschrieben.

Im Bereich der Fossa Piriformis wird der Markraum 2 des Femur 1 eröffnet. Danach wird der Manipuliermarknagel 10 in den Markraum 2 des Femur 1 vorgeschoben. Da der Manipuliermarknagel 10 unterdimensioniert ist, muss der Markraum 2 nicht erweitert werden. Das Einsetzen des Manipuliermarknagels 10 erfolgt mit Hilfe des Bügels 20, der ausserhalb des Körpers, lateral zu liegen kommt. Der gleiche Bügel 20 wird später auch für das Einsetzen des eigentlichen Marknagels verwendet. Neben der Einführfunktion wird der Bügel 20 auch als Zielvorrichtung für das Setzen der proximalen Verriegelungsschrauben durch den implantierten Marknagel 50 verwendet. Die dafür vorgesehenen Bohrungen 28 im bohrlehrenseitigen Ende 23 des Einschlagbügels 21 können gleichzeitig für die Montage der Bohrlehre 30 verwendet werden. Wie anhand der Fig. 2 erläutert ist die Bohrbüchse 33 der Bohrlehre 30 so ausgerichtet, dass sie in der Verlängerung, tangential neben dem Manipuliermarknagel 10 vorbei zielt. Dieser Offset, oder tangentiale Abstand, kann je nach dem Durchmesser des später einzusetzenden Marknagels 50 variiert werden. Normalerweise wird dieser tangentiale Abstand so gewählt, dass die durch die Bohrbüchse 33 eingesetzte Schraube, so nahe wie möglich neben dem später einzusetzenden Marknagel 50 zu liegen kommt.

Da die Bohrbüchse 33 drehbar gelagert ist, können die Schrauben in beliebiger Richtung gesetzt werden. Neben der freien Winkeleinstellung, kann die Schraubenlage nach distal oder proximal stufenlos verstellt werden. Je nach den anatomischen Verhältnissen können die Schrauben anterior oder posterior zum Nagel gesetzt werden. Die optimale Frakturkompression, kann durch paralleles Einbringen der Schrauben erzielt werden. Dieses Vorgehen wird durch die erfindungsgemässe Vorrichtung sehr einfach, da die Freiheitsgrade der Vorrichtung einzeln blockierbar sind (z.B. kann der Schraubenwinkel eingestellt und der Reiter 32 auf der Längsschiene 31 blockiert werden). Nach erfolgtem Einsetzen der Schraube, wird der Reiter 32 gelöst und nach distal oder proximal verschoben und wiederum blockiert.

Die Bohrbüchse 33 weist beispielsweise einen Aussendurchmesser von 11 mm und einen Innendurchmesser von 8 mm auf. Sie dient neben der Führung der diversen Bohrinstrumente und der Schraube beim Einsetzen, gleichzeitig als Gewebeschutzhülse, da je nach Indikation und Weichteilverhältnis, die Schrauben durch eine Stichinzision gesetzt werden. Um das Einsetzen der Bohrbüchse 33 durch die Inzision auf den Knochen zu erleichtern, wird vorzugsweise ein Trokar verwendet, der im Aussendurchmesser dem Innendurchmesser der Bohrbüchse 33 entspricht. Danach wird der Trokar entfernt und durch eine (zeichnerisch nicht dargestellte) intermediäre Bohrbüchse ersetzt, die den gleichen Aussendurchmesser besitzt, wie der Innendurchmesser der Bohrbüchse 33, nämlich 8 mm. Der Innendurchmesser dieser intermediär eingeschobenen Bohrbüchse ist je nach benötigtem Kerndurchmesser der eingesetzten Schraube variabel. Nach dem Bohren des Kernlochdurchmessers, wird die intermediär eingeschobene Bohrbüchse entfernt. Das Messen der benötigten Schraubenlänge, sowie das Einführen der Schraube, erfolgt durch die Bohrbüchse 33.
Sind die Schrauben gesetzt, wird die Bohrlehre 30 und der Manipuliermarknagel 10 entfernt. An Stelle des Manipuliermarknagels 10 kann jetzt der eigentliche Marknagel 50 eingesetzt werden. Je nach Situation muss der Markraum 2 zuerst auf den gewünschten Durchmesser aufgebohrt werden. Je nach Fraktursituation kann der Marknagel 50, durch zusätzliche quer zu seiner Längsachse eingebrachte Verriegelungsschrauben 51, gesichert werden, welche wiederum mit Hilfe des erfindungsgemässen Instrumentes gesetzt werden können.

In der Klinik kann es vorkommen, dass eine Schenkelhalsfraktur übersehen wird und dass der Chirurg eine solche erst bei der normalen Marknagelung erkennt. In einem solchen Fall, kann auch nach erfolgter Marknagelung, die erfindungsgemässe Vorrichtung auf den bereits implantierten Marknagel 50 montiert werden und die Schenkelhalsfraktur durch marknagelunabhängige Zugschrauben 43,44 sekundär fixiert werden. Dieses Vorgehen sollte jedoch nur im Notfall durchgeführt werden, da die Gefahr einer Durchblutungsstörung des Femurkopfes durch eine vorhergehende Marknagelung besteht.

## Patentansprüche

1. Chirurgisches Instrument zum Positionieren von osteosynthetischen Befestigungselementen, welches einen Marknagel (10;50) mit Aussendurchmesser D sowie einen U-förmigen Bügel (20) mit einem marknagelseitigen Ende (22) und einem bohrlehrenseitigen Ende (23) umfasst, wobei das marknagelseitige Ende (22) koaxial und in lösbarer Weise mit dem Marknagel (10;50) verbindbar ist und das bohrlehrenseitige Ende (23) eine daran befestigbare Bohrlehre (30) aufweist, dadurch gekennzeichnet, dass die Bohrlehre (30) mit einer Bohrbüchse (33) versehen ist, welche derart ausgerichtet ist, dass sie, in der Verlängerung, nur tangential neben dem Marknagel (10;50) mit dem vorgegebenen Aussendurchmesser D vorbeizielen kann.

2. Instrument nach Anspruch 1, dadurch gekennzeichnet, dass der vorgegebene Aussendurchmesser D maximal 12 mm beträgt.

3. Instrument nach Anspruch 1 oder 2, dadurch gekennzeichnet, dass die Bohrlehre (30) eine Längsschiene (31) mit Befestigungsmitteln (37) zur lösbaren Fixierung am bohrlehrenseitigen Ende (23) des Bügels (20) umfasst.

4. Instrument nach Anspruch 3, dadurch gekennzeichnet, dass die Bohrlehre (30) einen an der Längsschiene (31) befestigbaren und längsverschieblich angeordneten Reiter (32) umfasst, an dem seitlich die Bohrbüchse (33) drehbar gelagert ist.

5. Instrument nach einem der Ansprüche 1 - 4, dadurch gekennzeichnet, dass das marknagelseitigen Ende (22) des Bügels (20) als Hohlzylinderabschnitt ausgebildet ist und zur Verbindung mit dem Marknagel (10;50) Befestigungsmittel (12,24-27) aufweist, die einen konischen Gewindebolzen (24) mit einer vorderen Gewindepartie (26) und einer hinteren Gewindepartie (27), sowie eine Rändelmutter (25) umfassen, wobei der konische Gewindebolzen (24) durch das als Hohlzylinderabschnitt ausgebildete marknagelseitigen Ende (22) des Bügels (20) einführbar und mittels der Rändelmutter (25) und seiner beiden Gewindepartien (26,27) mit dem Marknagel (10;50) in lösbarer Weise verbindbar ist.

## Claims

1. Surgical instrument for positioning osteosynthetic attachment elements which comprises an intramedullary nail (10;50) with the outer diameter D as well as a U-shaped stirrup (20) with a medullary-nail end (22) and a drill-jig end (23), whereby the medullary-nail end (22) can be connected coaxially and separably with the intramedullary nail (10;50) and the drill-jig end (23) is provided with a drill-jig (30) connectable thereto,
**characterized in that**
the drill-jig (30) is provided with a jig bushing (33) which is oriented in such a way that, in its prolongation, it can only aim tangentially beside the intramedullary nail (10;50) with the given outer diameter D.

2. Instrument according to claim 1, characterized in that the given outer diameter D is 12 mm at most.

3. Instrument according to claim 1 or 2, characterized in that the drill jig (30) includes a longitudinal bar (31) with attachment devices (37) for the separable fixation at the drill-jig end (23) of the stirrup (20).

4. Instrument according to claim 3, characterized in that the drill jig (30) comprises an indicator (32) that can be attached to the longitudinal bar (31), and positioned to slide longitudinally, and at which jig bushing (33) is positioned laterally and rotatably.

5. Instrument according to one of the claims 1 - 4, characterized in that the medullary-nail end (22) of the stirrup (20) is designed as a hollow cylinder segment, and is provided with attachment devices (12, 24-27) for connection to the intramedullary nail (10;50), said attachment devices (12, 24-27) including a conical bolt (24) with a front threaded segment (26) and a rear threaded segment (27), as well as a hand nut (25), whereby the conical bolt (24) can be inserted through the medullary-nail end (22) of the stirrup (20), designed as a hollow cylinder segment, and by means of the hand nut (25) and its two threaded segments (26,27) is connectable separably with the intramedullary nail (10;50).

## Revendications

1. Instrument chirurgical en vue du positionnement d'éléments de fixation d'ostéosynthèse, qui comporte un clou médullaire (10; 50) d'un diamètre externe D ainsi qu'un étrier en forme de U (20) présentant une extrémité (22) située du côté du clou médullaire et une extrémité (23) située du côté du gabarit de perçage, tandis que l'extrémité (22) située du côté du clou médullaire peut être reliée coaxialement et de manière libérable au clou médullaire (10; 50), et que l'extrémité (23) située du côté du gabarit de perçage présente un gabarit de perçage (30) qui peut y être fixé, caractérisé en ce que le gabarit de perçage (30) est pourvu d'une douille de perçage (33) qui est orientée de telle sorte que son prolongement puisse uniquement viser tangentiellement à côté du clou médullaire (10; 50) présentant le diamètre externe D prédéterminé.

2. Instrument selon la revendication 1, caractérisé en ce que le diamètre externe prédéterminé D vaut au maximum 12 mm.

3. Instrument selon la revendication 1 ou 2, caractérisé en ce que le gabarit de perçage (30) comprend un axe allongé (31) avec des moyens de fixation (37) en vue de sa fixation libérable sur l'extrémité (23), située du côté du gabarit de perçage, de l'étrier (20).

4. Instrument selon la revendication 3, caractérisé en ce que le gabarit de perçage (30) comporte un coulisseau (32) disposé à coulissement longitudinal sur le rail allongé (31) et pouvant être fixé sur celui-ci, sur lequel la douille de perçage (33) est montée latéralement à rotation.

5. Instrument selon l'une quelconque des revendications 1-4, caractérisé en ce que l'extrémité (22), située du côté du clou médullaire, de l'étrier (20) est configurée comme partie cylindrique creuse, et présente en vue de sa liaison au clou médullaire (10; 50) des moyens de fixation (12, 24-27) qui comprennent un boulon fileté conique (24) présentant une partie filetée avant (26) et une partie filetée arrière (27) ainsi qu'un écrou cranté (25), tandis que le boulon fileté conique (24) peut être inséré à travers l'extrémité (22), située du côté du clou médullaire, configurée comme partie cylindrique creuse, de l'étrier (20), et peut être relié de manière libérable au clou médullaire (10; 50) au moyen de l'écrou cranté (25) et de ses deux parties filetées (26, 27).
